# EUROPEAN PATENT APPLICATION

(11) **EP 4 671 271 A1**
(43) Date of publication of application: **31.12.2025**
(21) Application number: 24785070.4
(22) Date of filing: 20.03.2024
(51) Int. Cl.: C07K 16/18, A61P 35/00, A61K 39/00

(54) **FC VARIANTS WITH IMPROVED HUMAN C1Q BINDING AFFINITY**

(30) Priority: 06.04.2023 KR 20230045116
(71) Applicant: Korea University Research and Business Foundation, Seoul 02841 (KR)
(72) Inventor: JUNG, Sang-Taek, Seoul 06217 (KR); LEE, Won-Ju, Seoul 02855 (KR); JO, Mi-Gyeong, Seoul 02717 (KR); KYUNG, Mun-Su, Gimpo-si Gyeonggi-do 10109 (KR)
(74) Representative: Isarpatent
(86) International application number: PCT/KR2024/003485
(87) International publication number: WO 2024/210362

(57) **Abstract**

The present invention relates to a glycosylated Fc variant with improved selective binding affinity to FcγRIIIa. Compared to conventional antibodies approved as antibody therapeutic agents, novel human antibody Fc domain variants of the present invention have reduced binding affinity to FcγRIIb, which is an immuno-inhibitory receptor, have improved binding affinity to FcγRIIIa (an increase in the A/I ratio), which is an immuno-activating receptor, thereby having a remarkably improved ability to induce ADCC, and have the effect of maximizing the immune action mechanism of therapeutic protein drugs, and thus can be effectively used in antibody drugs.

## Description

### [Technical Field]

The present disclosure relates to a glycosylated Fc variant with improved binding affinity to human C1q.

### [Background Art]

Protein therapeutic agents are widely used in clinical trials by rapidly replacing nonspecific low molecular compound therapeutics due to very high specificity for disease targets and low side effects and toxicity. Among protein therapeutic agents currently used in clinical trials, antibody therapeutic agents and Fc-fusion protein therapeutic agents fused with the Fc domain of an antibody are the main types. Therapeutic antibodies are considered as one of the most effective cancer therapies by exhibiting very high target specificity compared to conventional low molecular drugs, and having not only low biotoxicity and few side effects, but also an excellent blood half-life of about 3 weeks. One of the most important mechanisms of therapeutic antibodies is a mechanism for killing a target cell by recruiting complement molecules as a target antigen, and the Fc domain of the antibody plays a crucial role in complement molecule recruitment and complement-dependent cytotoxicity (CDC). In fact, large pharmaceutical companies and research institutes around the world are accelerating the research and development of therapeutic antibodies that specifically bind to cancer cells, including cancer-causing factors, to effectively remove the cancer cells. Companies for developing therapeutic antibody drugs mainly consist of pharmaceutical companies, such as Roche, Amgen, Johnson & Johnson, Abbott, and BMS. Particularly, Roche includes representative products of Herceptin, Avastin, Rituxan, and the like for anticancer treatment, and not only produces large profits, achieving sales of approximately $19.5 billion in the global market in 2012 with these three therapeutic antibodies, but also leads the global antibody drug market. Johnson & Johnson, which developed Remicade, is also growing rapidly in the global antibody market due to increased sales, and pharmaceutical companies such as Abbott and BMS are also known to have many therapeutic antibodies in the final stages of development. As a result, in the global pharmaceutical market, where low molecular drugs had been dominated, the low molecular drugs have been rapidly replaced with biopharmaceuticals including therapeutic antibodies that are specific to disease targets and have low side effects.

An antibody provides a linkage between the humoral and cellular immune systems, and a Fab region of the antibody recognizes an antigen, whereas an Fc domain region binds to a receptor (Fc receptor or FcR) for an antibody (immunoglobulin) on a cell that is differentially expressed by all immune competent cells and has a different mechanism depending on a type of FcγR expressed on the surface of the binding immune cell. An Fc receptor binding site on the Fc region of the antibody binds to the Fc receptor (FcR) on the cell, and the antibody binds to the Fc receptor on the cell surface through the Fc region to trigger a variety of important biological responses, including phagocy and destruction of antibody-coated particles, removal of immune complexes, lysis of antibody-coated target cells by killing cells (antibody-dependent cell-mediated cytotoxicity, or ADCC), release of inflammatory mediators, placental transfer and control of immunoglobulin production (Deo, Y.M. et al., Immunol. Today 18(3):127-135 (1997)). As such, the Fc domain plays a critical role in the recruitment of immune cells, and antibody-dependent cell-mediated cytotoxicity (ADCC) and antibody dependent cell-mediated phagocytosis (ADCP). In particular, the ADCC and ADCP functions, which are effector functions of the antibody, depend on interaction with Fc receptors present on the surfaces of many cells. Human Fc receptors are classified into five types, and the type of immune cell to be recruited is determined depending on which Fc receptor the antibody binds to. For example, the Fc domain of the antibody is responsible for the major therapeutic effect of therapeutic antibodies by inducing the effector functions of ADCC by binding to FcyRIIIa, ADCP by binding to FcyRI or FcyRIIa, and complement dependent cytotoxicity (CDC) by binding to C1q to have toxicity to the target antigen binding to the Fab region. To exhibit immune action mechanisms including CDC, the Fc domain of the antibody interacts with the C1 complex in the blood, and at this time, directly binds to a C1q molecule that constitutes the C1 complex to activate protease (C1r₂s₂) of the C1 complex. Then, a complement pathway is activated, ultimately forming a membrane attack complex on the target cell surface, resulting in the death of the target cell. In addition, anaphylatoxin and opsonin produced during the complement pathway recruit surrounding immune cells to induce complement-dependent cellular cytotoxicity (CDCC) and complement-dependent cellular phagocytosis (CDCP), thereby causing more diverse immune responses and efficiently eliminating target cells. The CDC of the antibody is highly dependent on the binding affinity between the Fc domain of the antibody and C1q, and IgG3 has higher C1q binding affinity and CDC activity than IgG1, but IgG3 has a disadvantage of having much lower FcRn binding affinity, which is required for the antibody half-life in the blood, and much lower protein A binding affinity than IgG1, which is required for a process of producing antibody drugs and Fc-fusion proteins.

Meanwhile, rituximab is a monoclonal antibody therapeutic agent that is commercialized for the purpose of treating tumors as a chimeric anti-CD20 monoclonal antibody and first approved from the US FDA. An antibody of the CD20 antigen in the early 1980s was a monoclonal antibody produced from mice, and had excellent reactivity with the antigen, but had the disadvantages of having a short in vivo half-life and causing antigenicity (HAMA, human antimouse antibody) due to a heteroantigen. To supplement the shortcomings of these mouse-derived antibodies and enhance biological effects, a chimeric antibody, rituximab, was synthesized through genetic recombination technology, in which a variable region for antigen recognition is mouse-derived and a constant region of the antibody is human-derived. The CD20 antigen is known to be involved in cell-cycle entry of B cell family or differentiation into B cells. The CD20 antigen is characterized to be suitable for use as a target molecule, due to characteristics of being not easily detached, modified, or incorporated from the cell surface. The CD20 antigen is widely distributed from a pre-B cell stage to an activated B cell stage in normal cells, but is not distributed in hematopoietic stem cells, plasma cells, and other family cells. In the case of lymphoma, the CD20 antigen is expressed in B-cell lymphoma and most of chronic lymphocytic leukemia and 50% of pre-B cell acute lymphoblastic leukemia to be an appropriate target for inducing tumor-specific immune responses using monoclonal antibodies (Maloney DG et al., IDEC-C2B8 (Rituximab) anti-CD20 monoclonal antibody therapy in patients with relapsed lowgrade non-Hodgkin's lymphoma. Blood, 90:2188-2195, 1997; McLaughlin P et al., Rituximab chimeric anti-CD20 monoclonal antibody therapy for relapsed indolent lymphoma: half of patients respond to a four-dose treatment program. J Clin Oncol, 16:2825-2833, 1998). However, there are some reports that rituximab is resistant in about half of low-grade lymphoma patients and about 60 to 70% of high-grade lymphoma patients, and complement-mediated cytotoxicity is reduced when expression of CD55 or CD59 is high. In addition, as a result of a study on the action mechanism of rituximab using an *in vivo* mouse model, it was shown that the efficacy of Rituxan was maintained in mouse models from which NK cells, PMNs, or T cells were deleted, but the efficacy of rituximab was completely eliminated in a C1q knockout mouse model, and thus it has been reported that complement activation is very important for therapeutic efficacy in antibody therapeutic agents (Di Gaetano et al 2003 J immunol). Therefore, attempts to improve the CDC of the antibody are urgently required to maximize the therapeutic effects of therapeutic antibodies and protein therapeutic agents.

### [Disclosure]

### [Technical Problem]

An object of the present disclosure is to provide a novel human antibody Fc domain variant.

In addition, another object of the present disclosure is to provide an antibody or immunologically active fragment thereof with improved binding affinity to C1q.

In addition, yet another object of the present disclosure is to provide a nucleic acid molecule encoding the Fc domain variant, or the antibody or immunologically active fragment thereof, a vector including the nucleic acid molecule, and a host cell including the vector.

In addition, still another object of the present disclosure is to provide a pharmaceutical composition for preventing or treating cancer.

In addition, still another object of the present disclosure is to provide a method for preparing a human antibody Fc domain variant.

In addition, still another object of the present disclosure is to provide a use of an antibody or immunologically active fragment thereof including the human antibody Fc domain variant to be used for preparation of an antibody therapeutic agent.

In addition, still another object of the present disclosure is to provide a use of an antibody or immunologically active fragment thereof for the prevention or treatment of cancer.

In addition, still another object of the present disclosure is to provide a method for treating cancer including administering an antibody or immunologically active fragment thereof in a pharmaceutically effective amount to a subject suffering from cancer.

### [Technical Solution]

To solve the above problem, one aspect of the present disclosure provides novel human antibody Fc domain variants with improved binding affinity to C1q.

In addition, another aspect of the present disclosure provides an antibody or immunologically active fragment thereof including the novel human antibody Fc domain variant.

In addition, yet another aspect of the present disclosure provides a nucleic acid molecule encoding the Fc domain variant, or the antibody or immunologically active fragment thereof, a vector including the nucleic acid molecule, and a host cell including the vector.

In addition, still another aspect of the present disclosure provides a pharmaceutical composition for preventing of treating cancer including the Fc domain variant, or the antibody or immunologically active fragment thereof as an active ingredient.

In addition, still another aspect of the present disclosure provides a method for preparing a human antibody Fc domain variant.

In addition, still another aspect of the present disclosure provides a use of an antibody or immunologically active fragment thereof including the human antibody Fc domain variant of the present disclosure to be used in the preparation of an antibody therapeutic agent.

In addition, still another aspect of the present disclosure provides a use of an antibody or immunologically active fragment thereof including the human antibody Fc domain variant of the present disclosure for the prevention or treatment of cancer.

In addition, still another aspect of the present disclosure provides a method for treating cancer including administering an antibody or immunologically active fragment thereof including the human antibody Fc domain variant of the present disclosure in a pharmaceutically effective amount to a subject suffering from cancer.

### [Advantageous Effects]

According to the present disclosure, the novel human antibody Fc domain variants exhibit superior C1q binding affinity to a wild-type human antibody Fc domain and previously known Fc variants with improved C1q binding affinity, have significantly increased CDC activity, and maintain FcRn binding affinity required for a blood antibody half-life and protein A binding affinity required for a Fc-fusion protein production process. Therefore, when the novel human antibody Fc domain variants are applied to antibody drugs and Fc-fusion protein drugs, there is an effect of dramatically improving therapeutic efficiency by maximizing CDC.

### [Description of Drawings]

FIG. 1 is a diagram illustrating results of SDS-PAGE analysis after expression and purification of hFcRn-GST.
FIG. 2 is a schematic diagram illustrating a strategy for preparing 30 types of rituximab-Fc variants using two types of backbone wild-type rituximab and rituximab-3HY clones.
FIGS. 3A and 3B are diagrams illustrating amino acid sequence information of Fc domains of wild-type IgG1 (IgG1 Fc), wild-type IgG3 (IgG3 Fc), IgG3-HY (IgG3-HY Fc), 113F variant (113F Fc), and 30 types of rituximab-Fc variants of the present disclosure.
FIG. 4 is a diagram illustrating results of SDS-PAGE analysis after expression and purification of rituximab-Fc variants.
FIG. 5 is a diagram illustrating results of ELISA analysis of C1q binding affinity of rituximab-Fc variants Sv1-1 to Sv1-15 of the present disclosure:
   RTX-1: Wild-type rituximab.
FIG. 6 is a diagram illustrating results of ELISA analysis of C1q binding affinity of rituximab-Fc variants Sv3-1 to Sv3-15 of the present disclosure:
   RTX-3(HY): Rituximab-3HY as R435H and F436Y variants of IgG3.
FIG. 7 is a diagram illustrating results of ELISA analysis of C1q binding affinity of selected rituximab-Fc variants with excellent C1q binding affinity based on the results of FIGS. 5 and 6.
FIG. 8 is a diagram illustrating results of ELISA analysis of C1q binding affinity of rituximab-Fc variants SV3-8, SV3-10 and SV3-11 with best C1q binding affinity based on the results of FIG. 7:
   RTX-WT: Wild-type rituximab; and
   RTX-113F: Rituximab including 113F Fc variant.
FIG. 9 is a diagram illustrating results of ELISA analysis of FcRn binding affinity of rituximab-Fc variants at pH 6.0 and 7.4:
   RTX-1: Wild-type rituximab; and
   RTX-3: Rituximab-3HY as R435H and F436Y variants of IgG3.
FIG. 10 is a diagram illustrating results of analyzing CDC activity of rituximab-Fc variants on a cancer cell line Daudi:
   RTX-WT: Wild-type rituximab;
   RTX-TL: Rituximab as T299L variant of IgG1;
   RTX-SV3-8: Rituximab-Fc variant SV3-8;
   RTX-SV3-10: Rituximab-Fc variant SV3-10; and
   RTX-SV3-11: Rituximab-Fc variant SV3-11.
FIG. 11 is a diagram illustrating results of analyzing CDC activity of rituximab-Fc variants on a cancer cell line Ramos:
   RTX-WT: Wild-type rituximab;
   RTX-TL: Rituximab as T299L variant of IgG1;
   RTX-SV3-8: Rituximab-Fc variant SV3-8;
   RTX-SV3-10: Rituximab-Fc variant SV3-10; and
   RTX-SV3-11: Rituximab-Fc variant SV3-11.
FIG. 12 is a schematic diagram illustrating a strategy for generating two types of libraries based on SV3-8 and SV3-10 variants.
FIG. 13 is a schematic diagram of a CHO cell display technology used in the present disclosure.
FIG. 14 is a diagram illustrating results of screening cell populations enriched through a total of 4 rounds for each library through screening using CHO cell display technology.
FIG. 15 is a diagram illustrating amino acid mutation sequence information of Fc variants EFTAE and KWES and Fc variants EFS11 and EFS16, which are finally selected as glycosylated Fc variants with further improved C1q binding affinity through a CHO cell Fc display system.
FIG. 16 is a diagram illustrating results of analyzing C1q binding affinity of rituximab-Fc variants RTX-EFS11 and RTX-EFS16 to a CD20⁺ targeted cancer cell line Daudi:
   RTX-IgG1: Wild-type IgGl;
   RTX-EFS11: Rituximab-Fc variant including EFS11 variant;
   RTX-EFS16: Rituximab-Fc variant including EFS16 variant;
   RTX-KWES: Rituximab-Fc variant including KWES variant; and
   RTX-EFTAE: Rituximab-Fc variant including EFTAE variant.
FIG. 17 is a diagram illustrating results of analyzing CDC activity of rituximab-Fc variants RTX-EFS11 and RTX-EFS16 on a CD20⁺ targeted cancer cell line Daudi:
   RTX-IgG1: Wild-type IgGl;
   RTX-EFS11: Rituximab-Fc variant including EFS11 variant;
   RTX-EFS16: Rituximab-Fc variant including EFS16 variant;
   RTX-KWES: Rituximab-Fc variant including KWES variant; and
   RTX-EFTAE: Rituximab-Fc variant including EFTAE variant.
FIG. 18 is a diagram illustrating results of analyzing FcγRIIIa binding affinity of rituximab-Fc variants RTX-EFS11 and RTX-EFS16:
   RTX-IgG1: Wild-type IgGl;
   RTX-EFS11: Rituximab-Fc variant including EFS11 variant;
   RTX-EFS16: Rituximab-Fc variant including EFS16 variant;
   RTX-KWES: Rituximab-Fc variant including KWES variant; and
   RTX-EFTAE: Rituximab-Fc variant including EFTAE variant.
FIG. 19 is a diagram illustrating results of analyzing FcyRIIa binding affinity of rituximab-Fc variants RTX-EFS11 and RTX-EFS16:
   RTX-IgG1: Wild-type IgGl;
   RTX-EFS11: Rituximab-Fc variant including EFS11 variant;
   RTX-EFS16: Rituximab-Fc variant including EFS16 variant;
   RTX-KWES: Rituximab-Fc variant including KWES variant; and
   RTX-EFTAE: Rituximab-Fc variant including EFTAE variant.

### [Best Mode]

Hereinafter, embodiments of the present disclosure will be described in detail with reference to the accompanying drawings. However, the following embodiments are presented as examples for the present disclosure, and when it is determined that the detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used in the present disclosure are terminologies used to properly express preferred examples of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be understood to imply the inclusion of stated elements, not the exclusion of any other elements.

All technical terms used in the present disclosure, unless otherwise defined, are used as the meaning as commonly understood by those skilled in the related art of the present disclosure. In addition, although preferred methods and samples are described herein, similar or equivalent methods and samples thereto are also included in the scope of the present disclosure. The contents of all publications described in the present disclosure as references are incorporated in the present disclosure.

Throughout the present specification, general one-letter or three-letter codes for naturally occurring amino acids are used, and generally allowed three-letter codes for other amino acids, such as *α*-aminoisobutyric acid (Aib) and N-methylglycine (Sar) are also used. The amino acids mentioned in the present disclosure as abbreviations are also described as follows according to the IUPAC-IUB nomenclature.

Alanine: A; Arginine: R; Asparagine: N; Aspartic acid: D; Cysteine: C; Glutamic acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y; and Valine: V.

In an aspect, the present disclosure relates to a human antibody Fc domain variant in which any one or more amino acids selected from the group consisting of amino acids at positions 249, 252, 268, 276, 326, 384, 392, 422, 435 and 436 numbered according to a Kabat numbering system in a wild-type human antibody Fc domain are substituted with sequences different from wild-type amino acids.

In one embodiment, the human antibody Fc domain variant of the present disclosure may include any one or more amino acid substitutions selected from the group consisting of 249N, 252I, 276N, 268Y, 276K, 326N, 384N, 384S, 392K, 392N, 422V, 422I, 435H, 435R, 436Y, and 436F.

In one embodiment, the antibody may be immunoglobulin (IgG), and may be IgA, IgM, IgE, IgD or IgG, or modifications thereof, more preferably IgG1 or IgG3 isotypes, and may be an anti-human epidermal growth factor receptor 2 (HER2) antibody, an anti-epidermal growth factor receptor (EGFR) antibody, or an anti-CD20 antibody, and more preferably trastuzumab, cetuximab or rituximab. Papain degradation of the antibody forms two Fab domains and one Fc domain, and in a human IgG molecule, a Fc domain is generated by papain degradation of the N-terminus at Cys 226 (Deisenhofer, Biochemistry 20: 2361-2370, 1981).

In one embodiment, the the human antibody Fc domain variant of the present disclosure may include any one or more amino acid substitutions selected from the group consisting of N276K, N384S, K392N, and V422I in a wild-type human IgG1 Fc domain, and the wild-type human IgG1 Fc domain may include an amino acid sequence represented by SEQ ID NO: 17.

In one embodiment, the human antibody Fc domain variant of the present disclosure may include any one or more amino acid substitutions selected from the group consisting of D249N, M252I, H268Y, K276N, K326N, S384N, N392K, I422V, R435H and F436Y in a wild-type human IgG3 Fc domain, and the wild-type human IgG3 Fc domain may include an amino acid sequence represented by SEQ ID NO: 18.

In one embodiment, the human antibody Fc domain variant of the present disclosure may have increased binding affinity to C1q compared to the wild-type human antibody Fc domain.

In one embodiment, the human antibody Fc domain variant of the present disclosure may have an increased effector function compared to the wild-type human antibody Fc domain. The effector function may be an Fc-mediated effector function selected from C1q-binding, complement activation, complement dependent cytotoxicity (CDC), antibody-dependent cellular cytotoxicity (ADCC), Fc-receptor binding including Fc-gamma receptor binding, protein A-binding, protein G-binding, antibody-dependent cell-mediated phagocytosis (ADCP), complement dependent cell-mediated cytotoxicity (CDCC), complement-enhanced cytotoxicity, opsonization, Fc-containing polypeptide internalization, target downmodulation, ADC uptake, induction of apoptosis, cell death, cell cycle arrest, and any combination thereof.

In one embodiment, the human antibody Fc domain variant of the present disclosure may increase complement dependent cytotoxicity (CDC) compared to the wild-type human antibody Fc domain. The human antibody Fc domain variant of the present disclosure may have increased CDC activity of 10% or more, 20% or more, 30% or more, 40% or more, 50% or more, 60% or more, 70% or more, 80% or more, 90% or more, or 100% or more compared to the wild-type Fc domain, or increased CDC activity of at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 60-fold, at least 70-fold, at least 80-fold, at least 90-fold, or at least 100-fold greater than the wild-type Fc domain.

In one embodiment, the human antibody Fc domain variant of the present disclosure may be an IgG3 variant and may have binding affinity to FcγRIIIa and FcγRIIa similar to or higher than that of wild-type IgG1.

In one embodiment, the wild-type human antibody Fc domain may include an amino acid sequence represented by SEQ ID NO: 17 or 18.

In one embodiment, the human antibody Fc domain variant of the present disclosure may include a heavy chain constant region domain 2 (C_{H}2) including any one selected from the group consisting of amino acid sequences represented by SEQ ID NOS: 1 to 8 and a heavy chain constant region domain 3 (C_{H}3) including any one selected from the group consisting of amino acid sequences represented by SEQ ID NOS: 9 to 16.

In an embodiment, the human antibody Fc domain variant of the present disclosure may include any one selected from the group consisting of amino acid sequences represented by SEQ ID NOS: 19 to 26.

In one embodiment, the human antibody Fc domain variant may include amino acid substitutions 276K and 384S, and may be Sv1-5 including amino acid substitutions N276K and N384S in a wild-type human IgG1 Fc domain, which may include a heavy chain constant region domain 2 including an amino acid sequence represented by SEQ ID NO: 1, a heavy chain constant region domain 3 including an amino acid sequence represented by SEQ ID NO: 9, or an Fc domain including an amino acid sequence represented by SEQ ID NO: 19.

In one embodiment, the human antibody Fc domain variant may include an amino acid substitution 384N, and may be Sv3-2 including an amino acid substitution S384N in a wild-type human IgG3 Fc domain, which may include a heavy chain constant region domain 2 including an amino acid sequence represented by SEQ ID NO: 2, a heavy chain constant region domain 3 including an amino acid sequence represented by SEQ ID NO: 10, or an Fc domain including an amino acid sequence represented by SEQ ID NO: 20.

The human antibody Fc domain variant may be Sv3-2 including an amino acid substitution S384N in a wild-type human IgG3 Fc domain,

In one embodiment, the human antibody Fc domain variant may include amino acid substitutions 384N and 392K, and may be Sv3-8 including amino acid substitutions S384N and N392K in a wild-type human IgG3 Fc domain, which may include a heavy chain constant region domain 2 including an amino acid sequence represented by SEQ ID NO: 3, a heavy chain constant region domain 3 including an amino acid sequence represented by SEQ ID NO: 11, or an Fc domain including an amino acid sequence represented by SEQ ID NO: 21.

In one embodiment, the human antibody Fc domain variant may include amino acid substitutions 392K and 422V, and may be Sv3-10 including amino acid substitutions N392K and I422V in a wild-type human IgG3 Fc domain, which may include a heavy chain constant region domain 2 including an amino acid sequence represented by SEQ ID NO: 4, a heavy chain constant region domain 3 including an amino acid sequence represented by SEQ ID NO: 12, or an Fc domain including an amino acid sequence represented by SEQ ID NO: 22.

In one embodiment, the human antibody Fc domain variant may include amino acid substitutions 276N, 384N and 392K, and may be Sv3-11 including amino acid substitutions K276N, S384N and N392K in a wild-type human IgG3 Fc domain, which may include a heavy chain constant region domain 2 including an amino acid sequence represented by SEQ ID NO: 5, a heavy chain constant region domain 3 including an amino acid sequence represented by SEQ ID NO: 13, or an Fc domain including an amino acid sequence represented by SEQ ID NO: 23.

In one embodiment, the human antibody Fc domain variant may include amino acid substitutions 276N, 384N and 422V, and may be Sv3-12 including amino acid substitutions K276N, S384N and I422V in a wild-type human IgG3 Fc domain, which may include a heavy chain constant region domain 2 including an amino acid sequence represented by SEQ ID NO: 6, a heavy chain constant region domain 3 including an amino acid sequence represented by SEQ ID NO: 14, or an Fc domain including an amino acid sequence represented by SEQ ID NO: 24.

In one embodiment, the human antibody Fc domain variant may include amino acid substitutions 268Y, 326N, 435H and 436Y, and may be EFS11 including amino acid substitutions H268Y, K326N, R435H and F436Y in a wild-type human IgG3 Fc domain, which may include a heavy chain constant region domain 2 including an amino acid sequence represented by SEQ ID NO: 7, a heavy chain constant region domain 3 including an amino acid sequence represented by SEQ ID NO: 15, or an Fc domain including an amino acid sequence represented by SEQ ID NO: 25.

In one embodiment, the human antibody Fc domain variant may include amino acid substitutions 249N, 252I, 268Y, 326N, 435H and 436Y, and may be EFS16 including amino acid substitutions D249N, M252I, H268Y, K326N, R435H and F436Y in a wild-type human IgG3 Fc domain, which may include a heavy chain constant region domain 2 including an amino acid sequence represented by SEQ ID NO: 8, a heavy chain constant region domain 3 including an amino acid sequence represented by SEQ ID NO: 16, or an Fc domain including an amino acid sequence represented by SEQ ID NO: 26.

In one embodiment, the human antibody Fc domain variant of the present disclosure may exhibit low binding affinity to FcRn at pH 7.0 to 7.8 similar to the wild-type human antibody Fc domain, and may be pH sensitive that exhibits high binding affinity to FcRn at pH 5.6 to 6.5.

In one embodiment, the Fc variant of the present disclosure may exhibit high binding affinity to FcRn at pH 5.6 to 6.5, and may be a weak acidic condition in the endosome, and pH 5.8 to 6.0. In the pH sensitive Fc variant of the present disclosure, the binding affinity to FcRn in the pH range may be the same or not substantially changed compared to the wild-type Fc domain.

In one embodiment, the Fc variant of the present disclosure may exhibit low binding affinity to FcRn at pH 7.0 to 7.8, and may be a normal pH range of blood and pH 7.2 to 7.6. The degree of dissociation of the Fc variant of the present disclosure from FcRn in the pH range may be the same or not substantially changed compared to the wild-type Fc domain.

In the present disclosure, variants including amino acid mutations in the human antibody Fc domain of the present disclosure are defined according to amino acid modifications constituting an Fc domain of a parent antibody, and conventional antibody numbering is followed by the EU index according to Kabat (Kabat et al., Sequence of proteins of immunological interest, 5th Ed., United States Public Health Service, National Institutes of Health, Bethesda, 1991).

As used in the present disclosure, the term "wild-type polypeptide" refers to an unmodified polypeptide that is modified later to produce a derivative. The wild-type polypeptide may be a polypeptide found in nature, or a derivative or manipulation of the polypeptide found in nature. The wild-type polypeptide may refer to a polypeptide itself, a composition including the wild-type polypeptide, or an amino acid sequence encoding the same. Accordingly, as used in the present disclosure, the term "wild-type antibody" refers to an unmodified antibody polypeptide in which amino acid residues are modified to produce a derivative. Interchangeably with the term, the "parent antibody" may be used to refer to an unmodified antibody polypeptide into which amino acid modifications are introduced to produce a derivative.

As used in the present disclosure, the term "amino acid modification/mutation" refers to substitution, insertion and/or deletion, preferably substitution of amino acids in a polypeptide sequence. As used in the present disclosure, the term "amino acid substitution" or "substitution" means that an amino acid at a specific position in the polypeptide sequence of the wild-type human antibody Fc domain is replaced with another amino acid. For example, an Fc variant including T299A substitution means that threonine, an amino acid residue at position 299 in the amino acid sequence of the wild-type antibody Fc domain, is replaced with alanine.

As used in the present disclosure, the term "Fc variant" means including a modification of one or more amino acid residues compared to the wild-type antibody Fc domain.

As used in the present disclosure, the term "Fc domain variant" may be used interchangeably with the "Fc variant".

In the present disclosure, the term "Fc domain", "Fc fragment", or "Fc region" forms an antibody with a Fab domain/fragment, and the Fab domain/fragment consists of a light chain variable region V_{L} and a heavy chain variable region V_{H}, a light chain constant region C_{L}, and a first heavy chain constant region C_{H}1, and the Fc domain/fragment consists of a second heavy chain constant region C_{H}2 and a third heavy chain constant region C_{H}3.

The Fc variant of the present disclosure includes one or more amino acid modifications compared to the wild-type antibody Fc domain (region or fragment), resulting in a difference in amino acid sequence. The amino acid sequence of the Fc variant according to the present disclosure is substantially homologous with the amino acid sequence of the wild-type antibody Fc domain. For example, the amino acid sequence of the Fc variant according to the present disclosure has about 80% or more, preferably about 90% or more, and most preferably about 95% or more homology compared to the amino acid sequence of the wild-type antibody Fc domain. The amino acid modifications may be performed genetically using molecular biological methods, or also performed using enzymatic or chemical methods.

The Fc variants of the present disclosure may be prepared by any method known in the art. In one embodiment, the human antibody Fc variant according to the present disclosure is used to form a nucleic acid in which a polypeptide sequence including a specific amino acid modification is encoded and then, if desired, cloned into a host cell, expressed, and assayed. Various methods therefor are described in the literature (Molecular Cloning-A Laboratory Manual, 3rd Ed., Maniatis, Cold Spring Harbor Laboratory Press, New York, 2001; Current Protocols in Molecular Biology, John Wiley & Sons).

The nucleic acid encoding the Fc variant according to the present disclosure may be inserted into an expression vector for protein expression. The expression vector generally includes a protein operably linked, i.e., functionally related to a control or regulatory sequence, a selectable marker, any fusion partner, and/or an additional element. Under appropriate conditions, the Fc variant according to the present disclosure may be produced by a method for inducing the protein expression by incubating a host cell transformed with a nucleic acid, preferably a nucleic acid-containing expression vector encoding the Fc variant according to the present disclosure. A variety of suitable host cells including mammalian cells, bacteria, insect cells, and yeast may be used, but are not limited thereto. Methods for introducing an exogenous nucleic acid into a host cell are known in the art and will vary depending on a host cell to be used. Preferably, the Fc variant according to the present disclosure is produced using E. coli having low production cost and high industrial value, as a host cell.

Accordingly, the scope of the present disclosure includes a method for preparing an Fc variant including incubating a host cell into which a nucleic acid encoding an Fc variant has been introduced, under conditions suitable for protein expression; and purifying or isolating the Fc variant expressed from the host cell. In addition, the antibodies including the polypeptide including the FC variant may be isolated and purified, and prepared by various methods known in the art. A standard purification method includes chromatography, electrophoresis, immunology, precipitation, dialysis, filtration, concentration, and chromatofocusing techniques. As known in the art, various natural proteins such as bacterial proteins A, G, and L bind to the antibody and these proteins may be used for purification. Often, purification by specific fusion partners may be enabled.

As used in the present disclosure, the term "FcRn" or "neonatal Fc receptor" refers to a protein that binds to an IgG antibody Fc domain, which is at least partially encoded by an FcRn gene. The FcRn may be derived from any organism, including humans, mice, rats, rabbits, and monkeys, but is not limited thereto. As known in the art, a functional FcRn protein includes two polypeptides, often referred to as light and heavy chains. The light chain is β-2-microglobulin, and the heavy chain is encoded by the FcRn gene. Unless otherwise stated herein, the FcRn or one FcRn protein refers to a complex of the FcRn heavy chain and the β-2-microglobulin.

In one aspect, the present disclosure relates to an antibody or immunologically active fragment thereof including the Fc domain variant of the present disclosure.

In an embodiment, the antibody may be a glycosylated antibody.

In one embodiment, the antibody or immunologically active fragment thereof of the present disclosure may have increased binding affinity to C1q compared to a wild-type human antibody.

In one embodiment, the antibody may be a polyclonal antibody, a monoclonal antibody, a minibody, a domain antibody, a bispecific antibody, an antibody mimetic, a chimeric antibody, an antibody conjugate, a human antibody, or a humanized antibody. The immunologically active fragment may be Fab, Fd, Fab', dAb, F(ab'), F(ab')₂, a single chain fragment variable (scFv), Fv, a single chain antibody, an Fv dimer, a complementarity determining region fragment, or a diabody of the antibody.

In one embodiment, the antibody of the present disclosure may be an anti-human epidermal growth factor receptor 2 (HER2) antibody, an anti-epidermal growth factor receptor (EGFR) antibody, or an anti-CD20 antibody, and may be trastuzumab, cetuximab, or rituximab.

In one embodiment, the trastuzumab may include a light chain including an amino acid sequence represented by SEQ ID NO: 27, the cetuximab may include a light chain including an amino acid sequence represented by SEQ ID NO: 28, and the rituximab may include a light chain including an amino acid sequence represented by SEQ ID NO: 29.

In one embodiment, in the antibody including the Fc variant of the present disclosure, a rituximab-Fc variant SV1-5 may include a heavy chain including an amino acid sequence represented by SEQ ID NO: 30.

In one embodiment, in the antibody including the Fc variant of the present disclosure, a rituximab-Fc variant SV3-2 may include a heavy chain including an amino acid sequence represented by SEQ ID NO: 31.

In one embodiment, in the antibody including the Fc variant of the present disclosure, a rituximab-Fc variant SV3-8 may include a heavy chain including an amino acid sequence represented by SEQ ID NO: 32.

In one embodiment, in the antibody including the Fc variant of the present disclosure, a rituximab-Fc variant SV3-10 may include a heavy chain including an amino acid sequence represented by SEQ ID NO: 33.

In one embodiment, in the antibody including the Fc variant of the present disclosure, a rituximab-Fc variant SV3-11 may include a heavy chain including an amino acid sequence represented by SEQ ID NO: 34.

In one embodiment, in the antibody including the Fc variant of the present disclosure, a rituximab-Fc variant SV3-12 may include a heavy chain including an amino acid sequence represented by SEQ ID NO: 35.

In one embodiment, in the antibody including the Fc variant of the present disclosure, a rituximab-Fc variant EFS11 may include a heavy chain including an amino acid sequence represented by SEQ ID NO: 36.

In one embodiment, in the antibody including the Fc variant of the present disclosure, a rituximab-Fc variant EFS16 may include a heavy chain including an amino acid sequence represented by SEQ ID NO: 37.

In one embodiment, the antibody of the present disclosure may include a light chain including any one selected from the group consisting of amino acid sequences represented by SEQ ID NOS: 21 to 23; and a heavy chain including any one selected from the group consisting of amino acid sequences represented by SEQ ID NOS: 30 to 37.

As used in the present disclosure, the term "glycosylation" refers to a processing method for transferring a glycosyl group to a protein. The glycosylation is performed by binding a glycosyl group to a serine, threonine, asparagine or hydroxylysine residue of a target protein by glycosyl transferase, and the glycosylated protein may not only be used as a component of living tissue, but also play an important role in cell recognition on the cell surface.

As used in the present disclosure, the term "trastuzumab-Fc variant", "cetuximab-Fc variant" or "rituximab-Fc variant" is introduced with the Fc variant of the present disclosure instead of an Fc region of a heavy chain of wild-type trastuzumab, cetuximab or rituximab. Therefore, the "trastuzumab-Fc variant", "cetuximab-Fc variant" or "rituximab-Fc variant" may be produced by preparing an expression vector into which the Fc variant of the present disclosure or a polypeptide including the Fc variant is introduced instead of the Fc region of the heavy chain of wild-type trastuzumab, cetuximab or rituximab, and preparing an expression vector into which the light chain of wild-type trastuzumab, cetuximab or rituximab is introduced, and transfected into an animal cell to be expressed.

The antibody may be isolated or purified by various methods known in the art. A standard purification method includes chromatography, electrophoresis, immunology, precipitation, dialysis, filtration, concentration, and chromatofocusing techniques. As known in the art, various natural proteins such as bacterial proteins A, G, and L bind to the antibody and these proteins may be used for purification. Often, purification by specific fusion partners may be enabled.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

The antibody or immunologically active fragment thereof of the present disclosure may be selected from the group consisting of animal-derived antibodies, chimeric antibodies, humanized antibodies, human antibodies, and immunologically active fragments thereof. The antibody may be produced recombinantly or synthetically.

The antibody or immunologically active fragment thereof may be isolated from a living body (not present in a living body) or may non-naturally occur, and for example, may be synthetically or recombinantly produced.

In the present disclosure, the "antibody" refers to a material produced by stimulation of an antigen in the immune system, and the type thereof is not particularly limited, and may be obtained naturally or non-naturally (e.g., synthetically or recombinantly). The antibody is advantageous for mass expression and production because of being very stable in vivo as well as ex vivo and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and has subclasses of gamma 1 (γ1), gamma 2 (γ2), gamma 3 (γ3), gamma 4 (γ4), alpha 1 (α1) and alpha 2 (α2). The light chain constant region has kappa (κ) and lambda (λ) types.

In the present disclosure, the term "heavy chain" is interpreted as a meaning including all of a full-length heavy chain including a variable region domain V_{H} including an amino acid sequence having a sufficient variable region sequence to impart specificity to an antigen, three constant region domains C_{H}1, C_{H}2 and C_{H}3 and hinges, and fragments thereof. In addition, the term "light chain" is interpreted as a meaning including all of a full-length light chain including a variable region domain V_{L} including an amino acid sequence having a sufficient variable region sequence to impart specificity to an antigen and a constant region domain C_{L}, and fragments thereof.

In the present disclosure, the term "Fc domain", "Fc fragment", or "Fc region" forms an antibody with a Fab domain/fragment, and the Fab domain/fragment consists of a light chain variable region V_{L} and a heavy chain variable region V_{H}, a light chain constant region C_{L}, and a first heavy chain constant region C_{H}1, and the Fc domain/fragment consists of a second constant region C_{H}2 and a third constant region C_{H}3 of the heavy chain.

In one aspect, the present disclosure relates to a nucleic acid molecule encoding the Fc domain variant of the present disclosure, or an antibody or immunologically active fragment thereof including the Fc domain variant.

In one aspect, the present disclosure relates to a nucleic acid molecule encoding the Fc domain variant of the present disclosure, or an antibody or immunologically active fragment thereof including the Fc domain variant.

In one aspect, the present disclosure relates to a vector including the nucleic acid molecule and a host cell including the vector.

The nucleic acid molecule of the present disclosure may be isolated or recombined, and includes not only DNA and RNA in single-stranded and double-stranded forms, but also complementary sequences corresponding thereto. The isolated nucleic acid is a nucleic acid that is isolated from surrounding genetic sequences present in the genome of a subject from which the nucleic acid is isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, DNA of a presequence or secretory leader is operably linked to DNA of the polypeptide when being expressed as a preprotein in the form before the polypeptide is secreted, a promoter or an enhancer is operably linked to a coding sequence when affecting the transcription of the polypeptide sequence, or a ribosome binding domain is operably linked to a coding sequence when disposed to promote the translation. In general, "operably linked" means that DNA sequences to be linked are located contiguously, and that the secretory leader exists contiguously in the same leading frame. However, the enhancer needs not to be contiguously located. The linkage is achieved by ligation at a convenient restriction enzyme site. When there is no site, synthetic oligonucleotide adapters or linkers are used according to conventional methods.

In the isolated nucleic acid molecule encoding the Fc domain variant of the present disclosure, or the antibody or immunologically active fragment thereof including the Fc domain variant, due to the degeneracy of codons or in consideration of codons preferred in an organism in which the isolated nucleic acid molecule is to be expressed, it will be well understood by those skilled in the art that various modifications may be made to a coding region within a range without changing the amino acid sequence of the Fc domain variant, or the antibody or immunologically active fragment thereof including the Fc domain variant to be expressed from the coding region, various modifications or changes may be made within a range without affecting the expression of the gene even in parts other than the coding region, and such modified genes are also included within the scope of the present disclosure. That is, as long as the nucleic acid molecule of the present disclosure encodes a protein having equivalent activity thereto, one or more nucleotides may be mutated by substitution, deletion, insertion, or a combination thereof, which are also included in the scope of the present disclosure. The sequence of such a nucleic acid molecule may be single- or double-stranded, and may be a DNA molecule or an RNA (mRNA) molecule.

The isolated nucleic acid molecule encoding the Fc domain variant of the present disclosure, or the antibody or immunologically active fragment thereof including the Fc domain variant according to the present disclosure may be inserted into an expression vector for protein expression. The expression vector generally includes a protein operably linked, i.e., functionally related to a control or regulatory sequence, a selectable marker, any fusion partner, and/or an additional element. In appropriate conditions, the Fc domain variant of the present disclosure, or the antibody or immunologically active fragment thereof including the Fc domain variant may be produced by a method for inducing the protein expression by culturing a host cell transformed with a nucleic acid, preferably an expression vector containing an isolated nucleic acid molecule encoding the Fc domain variant of the present disclosure, the antibody or immunologically active fragment thereof including the Fc domain variant. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, it is possible to produce E. coli, which has high industrial value due to low production cost, as a host cell.

The vector of the present disclosure may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer and may be variously produced according to the purpose. The promoter of the vector may be constitutive or inductive. The signal sequence may use a PhoA signal sequence, an OmpA signal sequence, etc. in the case of Escherichia sp. as a host, an α-amylase signal sequence, a subtilisin signal sequence, etc. in the case of Bacillus sp. as a host, an MFα signal sequence, a SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selective marker for selecting a host cell containing a vector and a replicable expression vector includes a replication origin.

In the present disclosure, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (e.g., bacteriophages), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994 etc.).

In one embodiment, when constructing the vector, expression regulatory sequences such as a promoter, a terminator, and an enhancer, sequences for membrane targeting or secretion, etc. are appropriately selected according to a type of host cell to produce the Fc domain variant, or the antibody or immunologically active fragment thereof including the Fc domain variant, and may be variously combined depending on a purpose.

In the present disclosure, the term "expression vector" refers to a vector including a nucleic acid sequence encoding at least a portion of a gene product to be transcribed. In some cases, an RNA molecule is then translated into a protein, a polypeptide, or a peptide. The expression vector may include various regulatory sequences. In addition to regulatory sequences that regulate transcription and translation, vectors and expression vectors may also include nucleic acid sequences that provide other functions.

In the present disclosure, the term "host cell" includes eukaryotes and prokaryotes, and refers to any transformable organism capable of replicating the vector or expressing a gene encoded by the vector. The host cell may be transfected or transformed by the vector, which means a process in which an exogenous nucleic acid molecule is delivered or introduced into the host cell.

In one embodiment, the host cell may be bacteria or animal cells, the animal cell line may be a CHO cell, a HEK cell or a NSO cell, and the bacteria may be E. coli.

In one aspect, the present disclosure relates to an antibody therapeutic agent or a pharmaceutical composition including an antibody or immunologically active fragment thereof including the Fc variant of the present disclosure.

In one embodiment, the Fc domain variant according to the present disclosure or a protein conjugate including the Fc domain variant may be bound and used with various bioactive polypeptides, such as cytokine, interleukin, interleukin binding protein, enzyme, antibody, growth factor, transcriptional regulator, blood factor, vaccine, structural protein, ligand protein or receptor, cell surface antigen, and receptor antagonist, and derivatives and analogues thereof, used for the purpose of treating or preventing human diseases.

In one aspect, the present disclosure relates to an antibody therapeutic agent in which an antibody or immunologically active fragment thereof of the present disclosure is conjugated to one or more therapeutic agents.

In one embodiment, the therapeutic agent may be a chimeric antigen receptor (CAR) cell therapy, an oncolytic drug, an immunotherapy agent, a cytotoxic agent, an angiogenesis inhibitor, a kinase inhibitor, a costimulatory molecule blocker, an adhesion molecule blocker, an anti-cytokine agent, an anti-CTLA-4 agent, an anti-PD-1 agent, an anti-PD-L1 agent, an anti-PD-L2 agent, a TNF-α cross-linking agent, a TRAIL cross-linking agent, an anti-CD27 agent, an anti-CD30 agent, an anti-CD40 agent, an anti-4-1BB agent, an anti-GITR agent, an anti-OX40 agent, an anti-TRAILR1 agent, an anti-TRAILR2 agent, tagretin, interferon-alpha, clobetasol, peginterferon, prednisone, romidepsin, bexarotene, methotrexate, triamcinolone cream, anti-chemokine, vorinostat, Gabapentin, cyclosporine, rapamycin, FK506, a detectable marker or reporter, a TNF antagonist, an antirheumatic agent, a muscle relaxant, narcotic, a non-steroid antiinflammatory drug (NSAID), analgesic, anesthetic, sedative, local anesthetic, neuromuscular blocker, antibacterial, psoriasis therapeutic agent, corticosteroid, anabolic steroid, erythropoietin, immunization, immunoglobulin, immunosuppressant, growth hormone, hormone replacement drug, radiopharmaceutical, antidepressant, antipsychotic, stimulant, asthma drug, beta agonist, inhaled steroid, epinephrine or analogue thereof, cytokine, cytokine antagonist, PD-1 antagonist, adenosine A2AR antagonist, CD73 inhibitor, CTLA-4 inhibitor, TIM-3 inhibitor, LAG-3 inhibitor, anthracycline, or combinations thereof.

In one aspect, the present disclosure relates to a pharmaceutical composition for preventing or treating cancer, including the human antibody Fc domain variant of the present disclosure, or the antibody or immunologically active fragment thereof including the Fc domain variant, as an active ingredient.

In one embodiment, the cancer may be any one selected from the group consisting of brain tumor, melanoma, myeloma, non-small cell lung cancer, oral cancer, liver cancer, gastric cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

In one embodiment, the composition of the present disclosure may further include an immunogenic apoptosis inducing agent. The immunogenic apoptosis inducing agent may be any one or more selected from the group consisting of anthracycline-based anticancer agents, taxane-based anticancer agents, anti-EGFR antibodies, BK channel agonists, Bortezomib, cardiac glycoside, cyclophosphamide-based anticancer agents, GADD34/PP1 inhibitors, LV-tSMAC, Measles virus, bleomycin, mitoxantrone, or oxaliplatin. The anthracycline-based anticancer agent may be daunorubicin, doxorubicin, epirubicin, idarubicin, pixantrone, sabarubicin, or valrubicin, and the taxane-based anticancer agent may be paclitaxel or docetaxel.

The pharmaceutical composition for preventing or treating cancer of the present disclosure is administered together with a chemical anticancer drug (anticancer agent) and the like to increase a cancer treatment effect of conventional anticancer agents through a cancer cell killing effect. Combined administration may be performed simultaneously or sequentially with the anticancer agent. Examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics such as dactinomycin (actinomycin D), plicamycin, and mitomycin C; plant alkaloids such as vincristine, vinblastine, etoposide, teniposide, topotecan, and iridotecan; and the like, but are not limited thereto.

In the present disclosure, the term "prevention" means all actions that inhibit or delay the occurrence, spread, and recurrence of cancer by administration of the pharmaceutical composition according to the present disclosure.

As used in the present disclosure, the term "treatment" refers to all actions that improve or beneficially change the death of cancer cells or the symptoms of cancer by administration of the composition of the present disclosure. Those skilled in the art to which the present disclosure pertains will be able to determine the degree of improvement, enhancement and treatment by knowing the exact criteria of disease for which the composition of the present disclosure is effective by referring to data presented by the Korean Academy of Medical Sciences, etc.

In the present disclosure, the term "therapeutically effective amount" used in combination with the active ingredient means an amount of pharmaceutically acceptable salt of the composition effective for preventing or treating a target disease, and the therapeutically effective amount of the composition of the present disclosure may vary depending on many factors, such as a method of administration, a target site, the condition of a patient, and the like. Accordingly, when used in the human body, a dose should be determined as an appropriate amount in consideration of both safety and efficiency. It is also possible to estimate an amount to be used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. As used in the present disclosure, the term "pharmaceutically effective amount" refers to an amount enough to treat the disease at a reasonable benefit/risk ratio applicable to medical treatment and enough not to cause side effects. The effective dose level may be determined according to factors including the health condition of a patient, the type and severity of cancer, the activity of a drug, the sensitivity to a drug, a method of administration, a time of administration, a route of administration, an excretion rate, duration of treatment, and drugs used in combination or simultaneously, and other factors well-known in the medical field. The composition of the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc and the like. The pharmaceutically acceptable additive according to the present disclosure is preferably included in an amount of 0.1 parts by weight to 90 parts by weight based on the composition, but is not limited thereto.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present disclosure may be administered parenterally (e.g., applied with injectable formulations intravenously, subcutaneously, intraperitoneally or topically) or orally according to a desired method, and the range of the dose may vary depending on the body weight, age, sex, and health condition of a patient, a diet, an administration time, an administration method, an excretion rate, the severity of a disease, and the like. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present disclosure relates to a method for preparing a human antibody Fc domain variant, including a) incubating a host cell including a vector including a nucleic acid molecule encoding the human antibody Fc domain variant of the present disclosure; and b) recovering a polypeptide expressed by the host cell.

In one aspect, the present disclosure relates to a method for preparing an antibody with increased CDC activity, including a) culturing a host cell including a vector including a nucleic acid molecule encoding the antibody or immunologically active fragment thereof of the present disclosure; and b) purifying the antibody expressed by the host cell.

In one embodiment, the purification of the antibody may include filtration, HPLC, anion exchange or cation exchange, high performance liquid chromatography (HPLC), affinity chromatography, or a combination thereof, and preferably affinity chromatography using Protein A.

In one aspect, the present disclosure relates to a use of an antibody or immunologically active fragment thereof including the human antibody Fc domain variant of the present disclosure to be used for the preparation of an antibody therapeutic agent.

In one aspect, the present disclosure relates to a use of an antibody or immunologically active fragment thereof including the human antibody Fc domain variant of the present disclosure for the prevention or treatment of cancer.

In one aspect, the present disclosure relates to a method for treating cancer including administering an antibody or immunologically active fragment thereof including the human antibody Fc domain variant of the present disclosure in a pharmaceutically effective amount to a subject suffering from cancer.

### [Modes]

Hereinafter, the present disclosure will be described in more detail through the following Examples. However, the following Examples are only intended to embody the contents of the present disclosure, and the present disclosure is not limited thereto.

### Example 1. Expression and purification of target substances

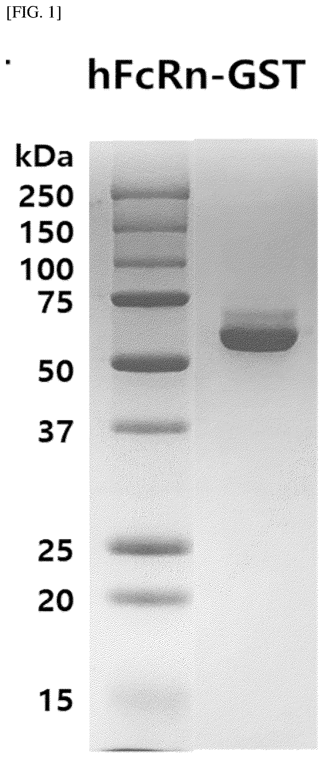

To discover Fc variants with improved C1q binding affinity and analyze FcRn binding affinity of the Fc variants, target substances were expressed and purified. Specifically, a pMAZ-hFcγRIIIa-158F-GST expression vector was fabricated, and polyethylenimine (PEI, Polyscience, 23966) and the expression vector gene were mixed in 30 ml of a Freestyle 293 expression culture medium (Gibco, 12338-018) at a ratio of 4 : 1, incubated at room temperature for 20 minutes, and then transfected into Expi293F animal cells cultured at a density of 2 × 10⁶ cells/ml the previous day and cultured for 7 days under conditions of 37°C, 125 rpm, and 8% CO₂. After culturing, the supernatant was collected by centrifugation at 6000×g for 15 minutes, and 30 ml of the culture supernatant was mixed with 12.5 ml of 25×PBS and filtered using a 0.2 µm bottle top filter (Corning, 430513). The culture medium transfected with the filtered pMAZ-hFcRn-GST was added with 500 µl of GST resin (Incospharm, 1101-3), stirred at 4°C for 16 hours, and then packed in a disposable polypropylene column to recover a resin. Thereafter, the resin was washed with 100 ml 1×PBS (pH 7.4) and eluted with 50 mM Tris-HCl and 10 mM GSH buffer (pH 8.0) diluted in 4 ml of 1×PBS. The buffer was exchanged with 1×PBS (pH 7.4) using Amicon Ultra-4 centrifugal filter units 3K (Merck Millipore, UFC800324), and as a result, it was confirmed through SDS-PAGE gel that hFcRn-GST was successfully purified with high purity (FIG. 1).

### Example 2. Design and discovery of Fc variants with improved binding affinity to C1q

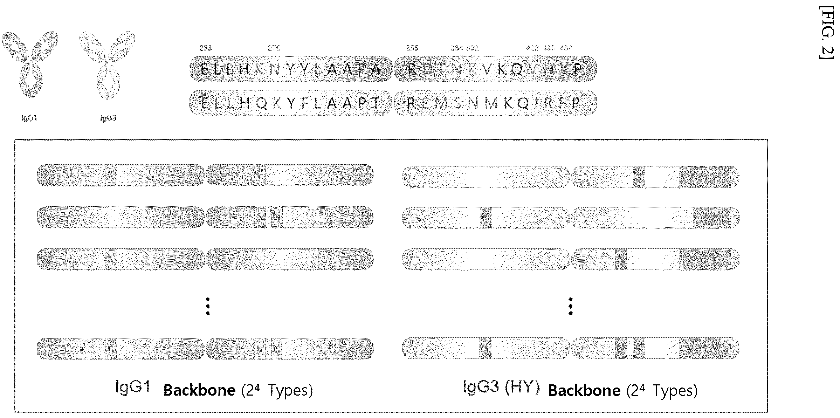

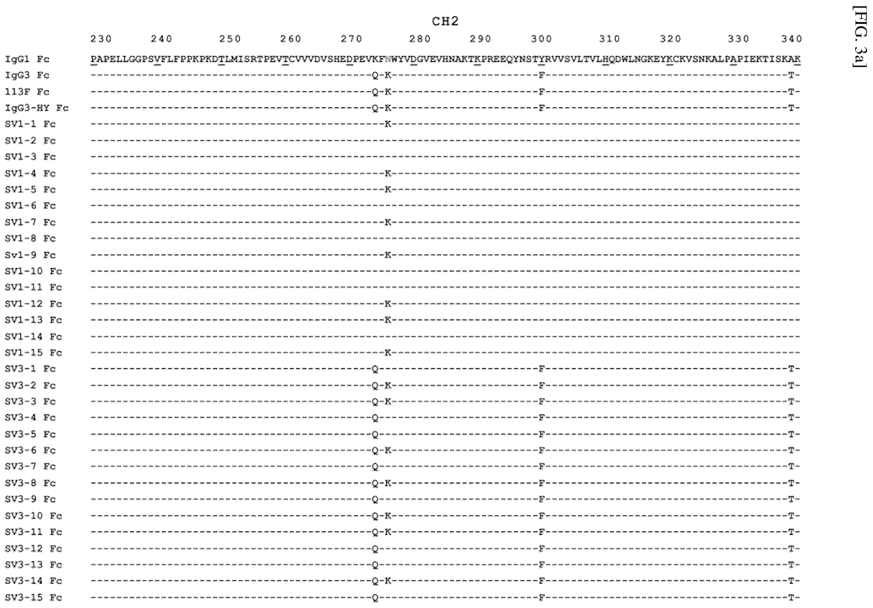

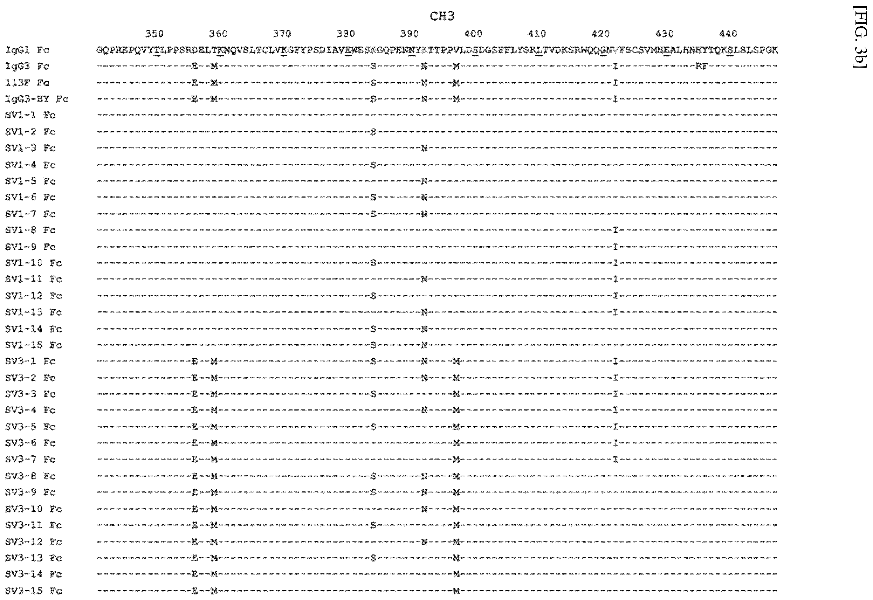

Since IgG3 had a short blood half-life due to very low FcRn binding affinity compared to IgG1, and inefficient production due to a long and flexible hinge, wild-type rituximab (IgG1) and rituximab-3HY (R435H and F436Y variants of IgG3) modified so that a region directly binding to FcRn and a hinge had the same sequences as IgG1 were fabricated as two backbones (FIG. 2). By identifying different amino acid sequences between IgG1 and IgG3 and selecting amino acid sequences (IgG1: N276, N384, K392, and V422; and IgG3: K276, S384, N392, and I422) to be expected to be important for C1q binding, and performing shuffling to introduce mutations in a random combination into each backbone prepared above, 30 types of rituximab Fc variants were prepared, including 15 types of rituximab Fc variants Sv1-1 to Sv1-15 in which an amino acid of IgG1 was substituted with an amino acid type K276, S384, N392, or I422 of IgG3 and 15 types of rituximab Fc variants Sv3-1 to Sv3-15 in which an amino acid of IgG3 was substituted with an amino acid type N276, N394, K392, or V422 of IgG1 (FIGS. 2, and 3A and 3B).

### Example 3. Expression and purification of rituximab-Fc variants with improved binding affinity to C1q

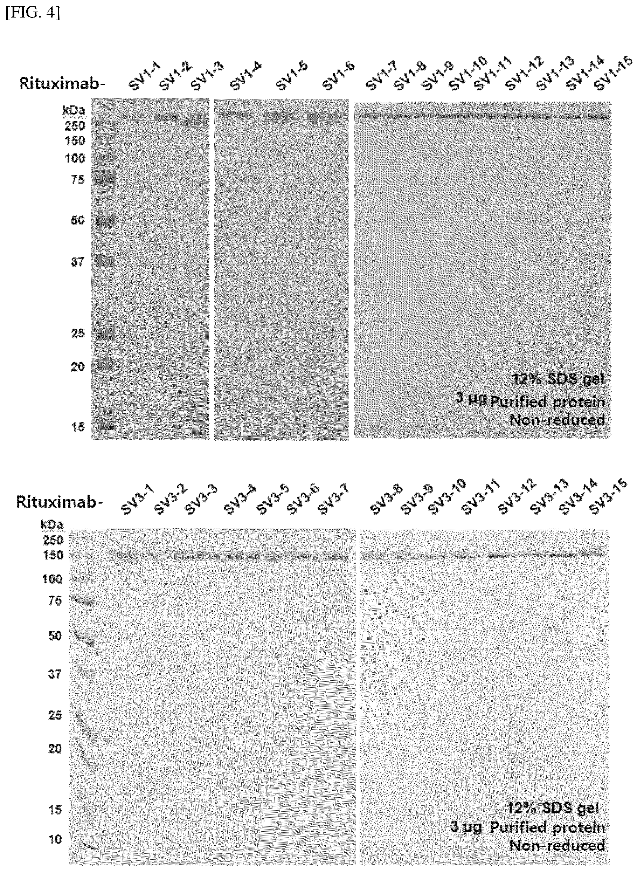

In order to express and purify each of the 30 types of rituximab Fc variants prepared in Example 2 above, pMAZ-Rituximab-HC was prepared as an expression vector for the 30 types of rituximab Fc variants. Heavy chain genes and light chain genes of the respective variants were first mixed in 3 ml of a Freestyle 293 expression culture medium (Gibco, 12338-018) at a 1 : 1 ratio, and mixed at a ratio of PEI : variant gene = 4 : 1, left at room temperature for 20 minutes, and then transfected into Expi293F cells subcultured at a density of 2 × 10⁶ cells/ml the previous day. Thereafter, the transfected cells were incubated in a CO₂ shaking incubator for 7 days under conditions of 37°C, 125 rpm, and 8% CO₂, and centrifuged at 6000×g for 15 minutes to collect only the supernatant. Thereafter, 30 ml of the collected culture supernatant was mixed with 1.25 ml of 25×PBS and then filtered using a 0.2 µm bottle top filter (Corning, 430513). The filtered culture medium was added with 100 µl of Protein A resin, stirred at 4°C for 16 hours, and then packed in a disposable polypropylene column to recover a resin. The resin was washed with 5 ml of 1×PBS (pH 7.4), eluted with 3 ml of 100 mM glycine (pH 2.7) buffer, and then neutralized using 1 M Tris-HCl (pH 8.0). The buffer was exchanged with 1×PBS (pH 7.4) using Amicon Ultra-4 centrifugal filter units 3K (Merck Millipore, UFC800324), and as a result, it was confirmed through SDS-PAGE gel that the antibody rituximab-Fc variants were successfully purified with high purity (FIG. 4).

### Example 4. Analysis of C1q binding affinity of rituximab-Fc variants

The C1q binding affinity of the rituximab-Fc variants expressed and purified in Example 3 above was analyzed using ELISA. Specifically, 50 µl each of the rituximab-Fc variants diluted in 4 µg/ml of 0.05 M Na₂CO₃ (pH 9.6) was incubated and immobilized in a flat bottom polystyrene High Bind 96 well microplate (Costar, 3590) at 4°C for 16 hours, and then blocked with 100 µl of 4% skim milk (GenomicBase, SKI400) at room temperature for 2 hours. The microplate was washed four times with 180 µl of 0.05% PBST, and then 50 µl of C1q (Quidel, A400) serially diluted with 1% skim milk was dispensed into each well and reacted at room temperature for 1 hour. Each well was washed and then reacted with 50 µl of C1q antibody-HRP (Invitrogen, PA1-84324) at room temperature for 1 hour and washed. The wells were added with 50 µl each of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was measured using an Epoch microplate spectrophotometer (BioTek).

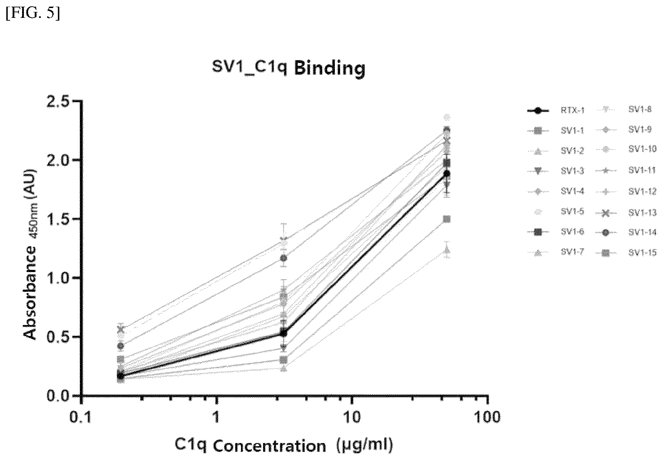

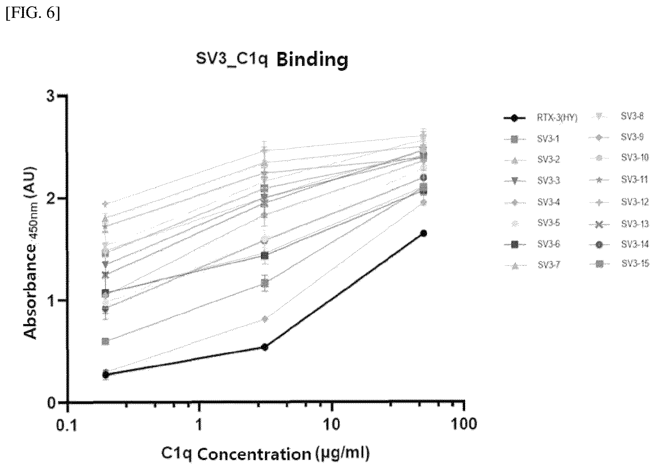

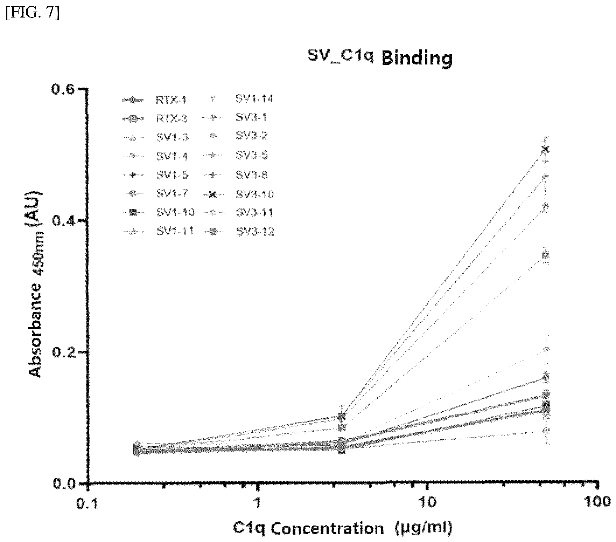

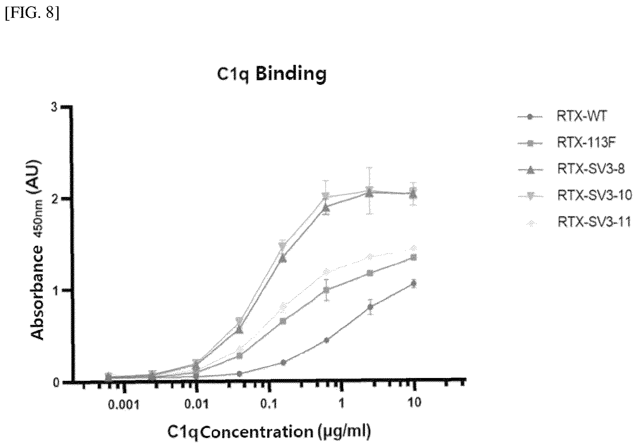

As a result, it was confirmed that many of the glycosylated rituximab Fc variants discovered in the present disclosure had significantly improved C1q binding affinity compared to wild-type rituximab or rituximab into which a conventional 113F variant was introduced (FIGS. 5 to 8), and particularly, three types of rituximab Fc variants SV3-8, SV3-10, and SV3-11 exhibited the highest C1q binding affinity.

### Example 5. Analysis of FcRn binding affinity of rituximab-variants

ELISA analysis was performed to determine pH-dependent human FcRn binding affinity of rituximab-Fc variants, including the discovered Fc variants SV3-8, SV3-10, and SV3-11. Specifically, 50 µl each of hFcRn-GST diluted at 4 µg/ml in 0.05 M Na₂CO₃ (pH 9.6) was incubated and immobilized in a flat bottom polystyrene High Bind 96 well microplate (Costar, 3590) at 4°C for 16 hours, and then blocked at room temperature for 2 hours with 4% skim milk (GenomicBase, SKI400) diluted in 100 µl of 1×PBS (pH 7.4). The microplate was washed four times with 150 µl of 0.05% PBST (pH 7.4), 50 µl each of wild-type rituximab (RTX-1), rituximab-3HY (RTX-3), and rituximab-Fc variants SV3-8, SV3-10, and SV3-11 diluted at 20 µg/ml in 1% skim milk diluted in 1× PBS (pH 7.4) were dispensed into each well and reacted at room temperature for 1 hour. Each well was washed four times with 100 µl of 0.05% PBST (pH 6.0/pH 7.4), and then antibody reaction was performed at room temperature for 1 hour using 50 µl of HRP-Protein L (GenScript, M00098) diluted in 1% skim milk (pH 6.0/pH 7.4) diluted in 1×PBS, and then washed four times with 100 µl of 0.05% PBST (pH 6.0/pH 7.4). The wells were added with 50 µl each of a 1-Step Ultra TMB-ELISA substrate solution (Thermo Fisher Scientific, 34028) to develop color, and then added with 50 µl each of 2 M H₂SO₄ to terminate the reaction. Thereafter, absorbance was measured using an Epoch microplate spectrophotometer (BioTek).

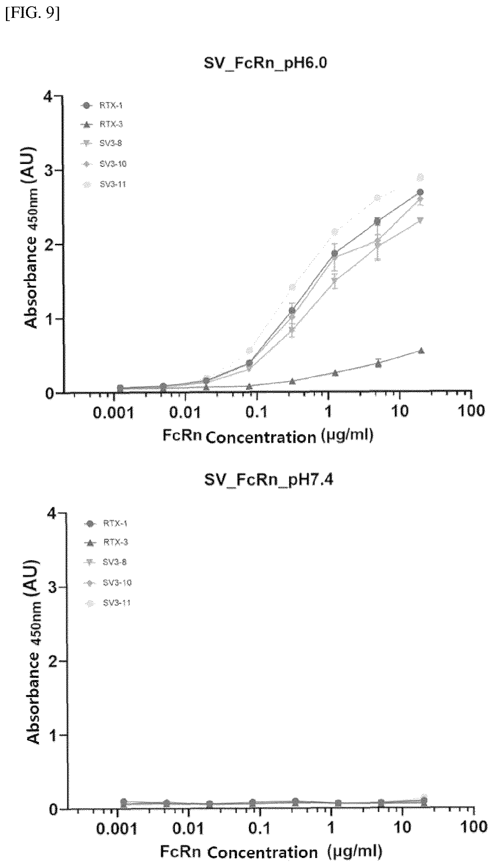

As a result, it was confirmed that the rituximab-Fc variants maintained pH-dependent FcRn binding similarly to wild-type rituximab (FIG. 9).

### Example 6. Analysis of CDC activity of rituximab-variants

To determine the complement-dependent cytotoxicity (CDC) activity of rituximab-Fc variants including the discovered Fc variants SV3-8, SV3-10, and SV3-11, CDC analysis was performed. Specifically, RPMI-1640 (Gibco, 11875119) and complement sera human (Sigma-Aldrich, S1764-5X1ML) were mixed in a 4 : 1 ratio to prepare a 20% human serum complement, and a targeted cancer cell line Ramos or Daudi was dispensed into the prepared 20% human serum complement, and 5 × 10⁵ cells were dispensed at 100 µl per well in a V-bottom 96-well cell culture plate (Corning, 3894). Thereafter, 20 µl each of wild-type rituximab RTX-WT, a rituximab-Fc variant RTX-1 (rituximab, a T299L variant of IgG1), SV3-8, SV3-10, and SV3-11 prepared in the 20% human serum complement were dispensed into each well to be the final concentration of the rituximab-Fc variants of 1.25 µg/ml or 5 µg/ml, respectively. Each well was incubated for 2 hours under 37°C and 5% CO₂ conditions, and then 120 µl each of FITC Annexin V and 7-AAD (BioLegend, 640922) diluted at 0.5 µg/ml of lysed cancer cells in Annexin V binding buffer (Biolegend, 422201) were dispensed to each well, and incubated at room temperature for 20 minutes. Thereafter, the supernatant was discarded after centrifugation at 400×g for 5 minutes, and each well was treated with 100 µl of Annexin V binding buffer to lyse the cells, and then each well was added with 100 µl of a fixative prepared by diluting 6% formaldehyde (Sigma-Aldrich, 252549) in Annexin V binding buffer, mixed well by pipetting, and incubated at room temperature for 15 minutes. The supernatant was discarded after centrifugation at 400×g for 5 minutes, and the cells were lysed with 500 µl of annexin V binding buffer, and then FITC and 7-AAD fluorescence signals were analyzed using FACS lyric to calculate cytotoxicity.

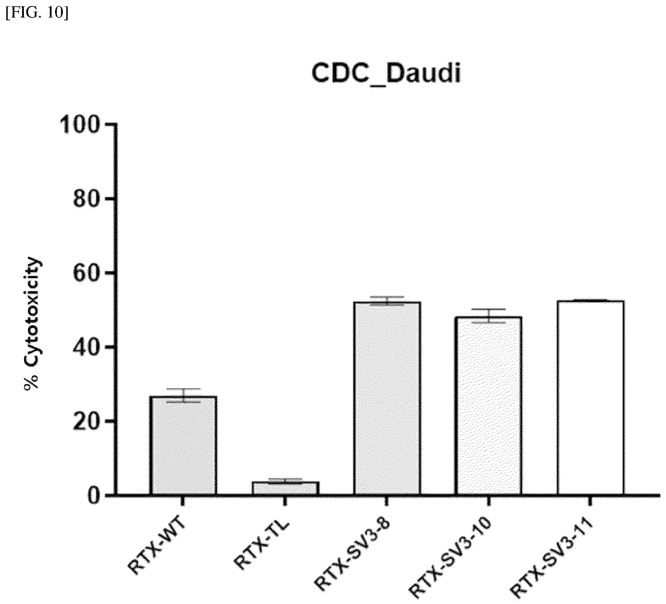

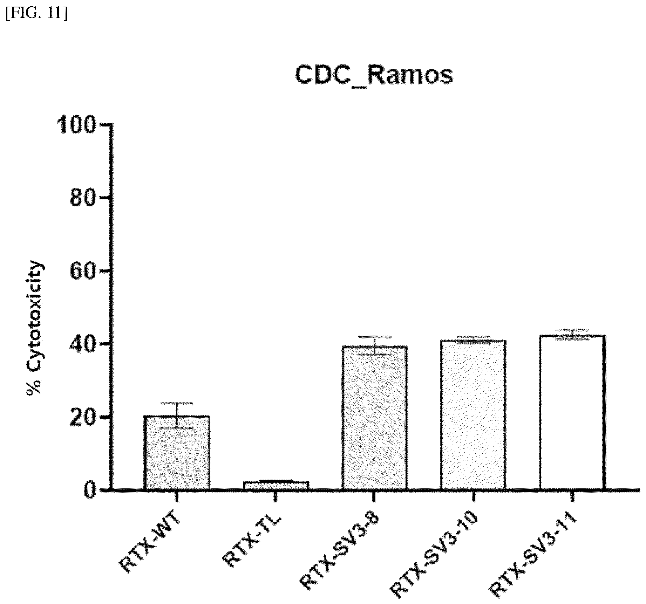

As a result, it was confirmed that all of the rituximab-Fc variants of the present disclosure exhibited significantly improved CDC activity compared to wild-type rituximab (FIGS. 10 and 11).

### Example 7. Construction of Fc variant library with improved C1q binding affinity

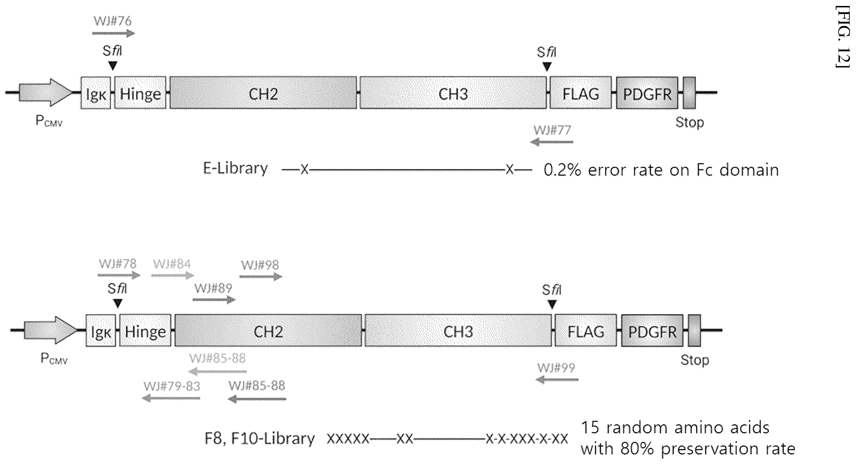

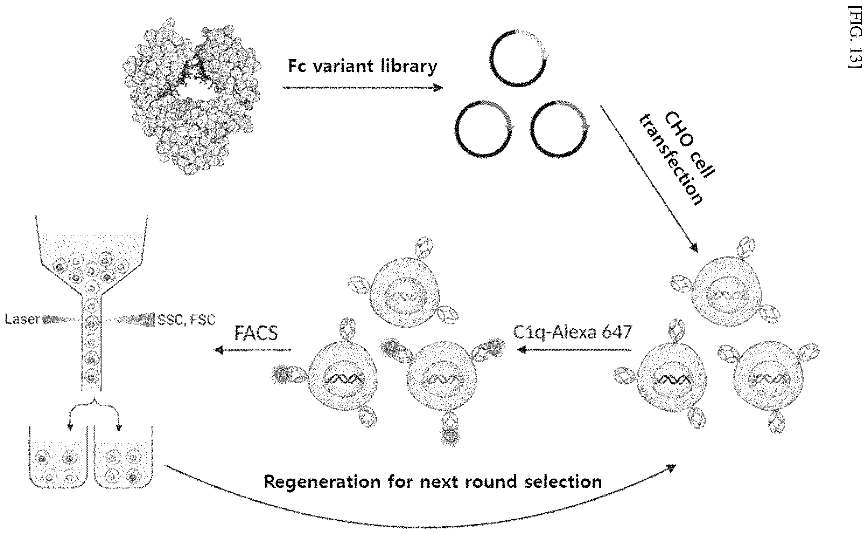

In order to engineer glycosylated Fc through a CHO cell Fc display system and select glycosylated Fc variants with further improved C1q binding affinity than the rituximab-Fc variants discovered in Examples 1 to 6 above, a library was constructed by introducing random mutations based on a SV3-8 variant (S384N and N392K mutations based on the IgG3 Fc domain) and a SV3-10 variant (N392K and I422V mutations based on the IgG3 Fc domain). Specifically, two types of libraries were classified according to a mutation introduction region: 1) E-Library: Constructed by introducing random mutations through error-prone PCR to entire Fc region of SV3-8 or SV3-10 variant; and 2) F-Library: Constructed by performing 1R sorting on F8-Library and F10-Library constructed by introducing random mutations intensively only in a region directly involved in C1q binding among the Fc regions of SV3-8 and SV3-10 variants, respectively, and then combining F8-Library and F10-Library (FIG. 12). The library genes were co-transfected with FLP expression plasmid in the same manner as a method for producing a stable Fc expression CHO cell line, and then a selection process using a hygromycin-B medium was performed to produce CHO cell lines stably expressing the glycosylated Fc variant library, respectively (FIG. 13).

### Example 8. Final selection of Fc variants with improved C1q binding affinity

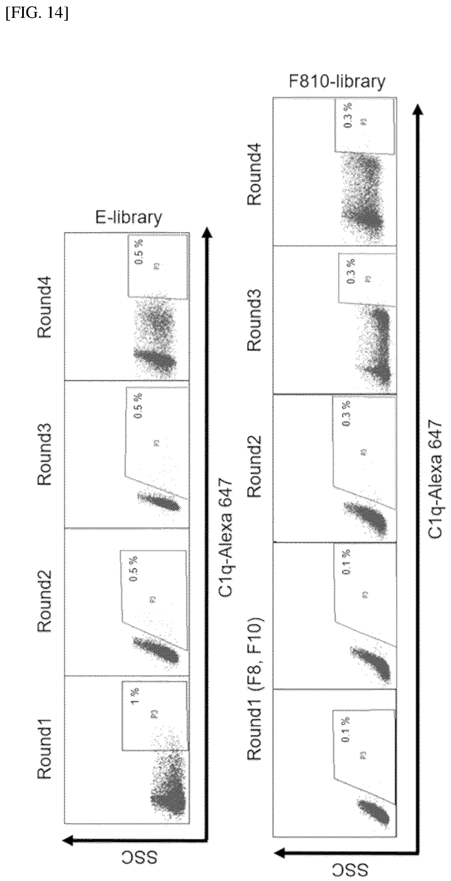

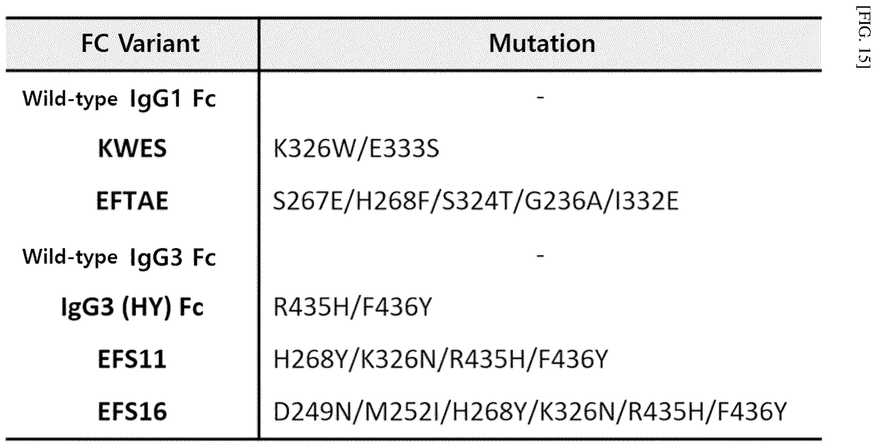

In order to select Fc with improved C1q binding affinity using the CHO cell line prepared using the CHO cell Fc display system in Example 7 above, 4R FACS sorting on each of two libraries E and F was performed by inducing binding of C1q-Alexa647 to a CHO cell line stably expressing the glycosylated Fc variant library, and then monitoring C1q binding. Through this, a cell population to be expected to exhibit excellent C1q binding affinity was selected, and the selected glycosylated Fc variant-expressing CHO cells were recovered through genomic DNA prep., and then engineered Fc variants were selected by identifying base sequences (FIG. 14). After analyzing the effects of mutations included in the selected Fc variants on Fc-C1q binding, Fc variants EFS11 (H268Y, K326N, R435H, and F436Y based on the IgG3 Fc domain) and EFS16 (D249N, M252I, H268Y, K326N, R435H, and F436Y based on the IgG3 Fc domain) were finally selected as Fc variants with further improved C1q binding affinity through the most optimal mutation combination (FIG. 15).

### Example 9. Analysis of C1q binding affinity of rituximab-Fc variants including finally selected Fc variants

In order to evaluate the C1q binding affinity of a rituximab-Fc variant RTX-EFS11 including a EFS 11 variant and a rituximab-Fc variant RTX-EFS16 including a EFS16 variant under physiochemical conditions, a CD20⁺ targeted cancer cell line Daudi was suspended in 1×PBS, and 1 µg/ml of C1q reacted with 1.25 µg/ml of final concentrations of RTX-EFS11 and RTX-EFS16 at 37°C for 30 minutes, respectively. Thereafter, the cells were centrifuged, washed once with 1% BSA-PBS, mixed with FITC anti-C1q (Invitrogen, PA1-29720) at a ratio of 50 : 1, stained on ice for 30 minutes, washed, and then analyzed using FACS Lyric (BioTek). At this time, for comparison, KWES variants (K326W and E333S based on IgG1) developed by Amgen and EFTAE variants (S267E, H268F, S324T, G236A, and I332E based on IgG1) developed by Xencor were used (FIG. 15).

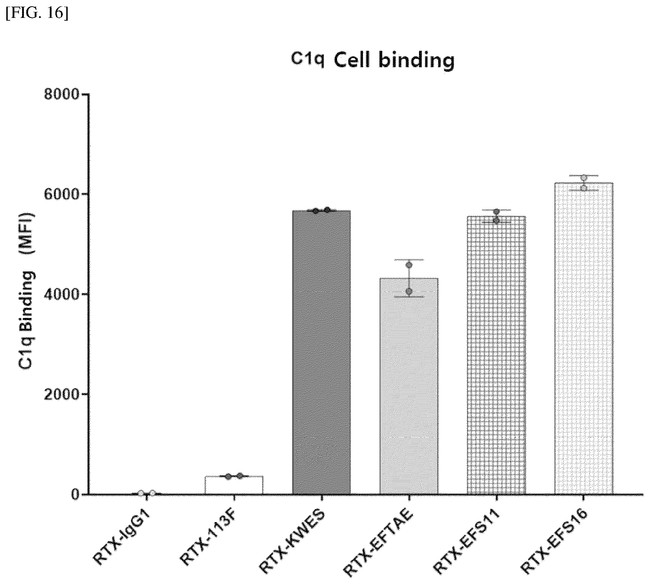

As a result, it was confirmed that rituximab including the Fc variant EFS11 and rituximab including EFS16, which were finally selected in the present disclosure, had improved C1q binding affinity compared to conventional rituximab including a KWES variant and rituximab including an EFTAE variant (FIG. 16).

### Example 10. Analysis of CDC activity of rituximab-Fc variants including finally selected Fc variants

To confirm the complement-dependent cytotoxicity (CDC) activity of the finally selected rituximab-Fc variant RTX-EFS11 including the EFS 11 variant and rituximab-Fc variant RTX-EFS16 including the EFS16 variant, the CDC analysis was performed using the method described in Example 6 above, and for activity comparison, KWES variants (K326W and E333S based on IgG1) developed by Amgen and EFTAE variants (S267E, H268F, S324T, G236A, and I332E based on IgG1) developed by Xencor were used.

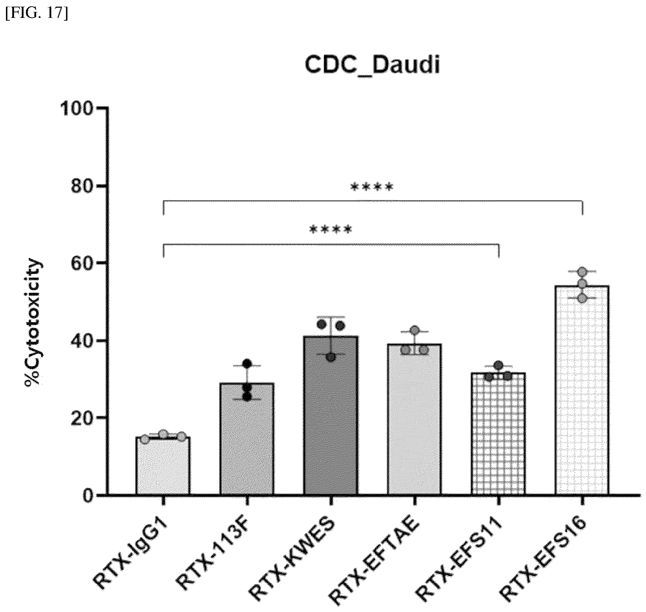

As a result, it was shown that rituximab including the Fc variant EFS11 and rituximab including EFS16, which were finally selected in the present disclosure, had improved CDC activity compared to conventional rituximab including the KWES variant and rituximab including the EFTAE variant (FIG. 17).

### Example 11. Analysis of FcγRIIIa and FcγRIIa binding affinity of rituximab-Fc variants including finally selected Fc variants.

To confirm the FcγRIIIa and FcyRIIa binding affinity of the finally selected rituximab-Fc variant RTX-EFS11 including the EFS11 variant and rituximab-Fc variant RTX-EFS16 including the EFS16 variant, ELISA analysis was performed. Specifically, 50 µl each of FcγRs-GSTs (FcγRIIIa-158V-GST and FcγRIIa-GST) diluted to 4 µg/ml in 0.05 M Na₂CO₃ (pH 9.6) were incubated and immobilized on a flat bottom polystyrene High Bind 96 well microplate (Costar, 3590) at 4°C for 16 hours, respectively, and then blocked with 100 µl of 4% skim milk (GenomicBase, SKI400) at room temperature for 2 hours. The microplate was washed four times with 180 µl of 0.05% PBST, and then 50 µl each of glycosylated rituximab-Fc variants RTX-EFS11 and RTX-EFS16 serially diluted with 1% skim milk were dispensed into each well and reacted at room temperature for 1 hour. After washing, antibody reaction was performed at room temperature for 1 hour using 50 µl of HRP-Protein L (GenScript, M00098), and the washing process was performed. The wells were added with 50 µl each of a 1-Step Ultra TMB-ELISA substrate solution (Thermo Fisher Scientific, 34028) to develop color, and then added with 50 µl each of 2 M H₂SO₄ to terminate the reaction, and then absorbance was measured using an Epoch microplate spectrophotometer (BioTek). At this time, wild-type IgG1 RTX-IgG1, rituximab-KWES variant, and rituximab-EFTAE variant were used for comparison.

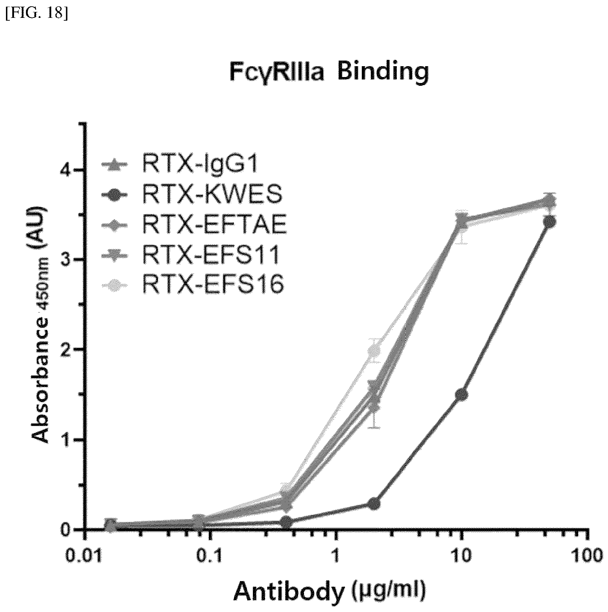

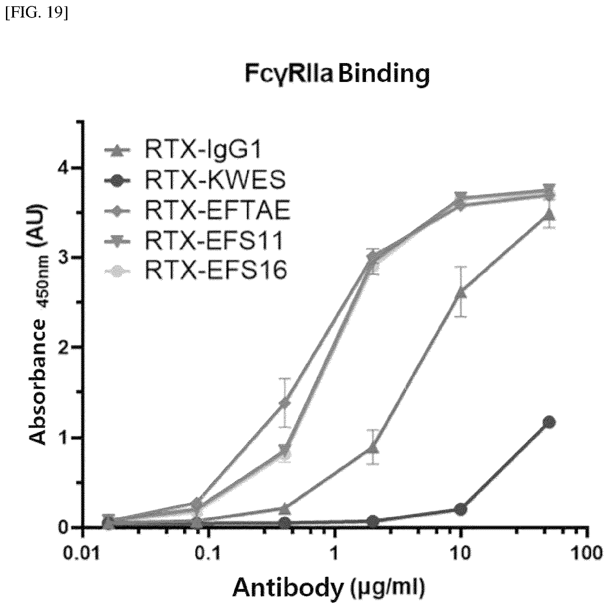

As a result, it was shown that rituximab including the Fc variant EFS11 and rituximab including EFS16, which were finally selected in the present disclosure, had significantly high FcγRIIa binding affinity and similar FcγRIIIa binding affinity compared to wild-type IgG1, and had superior binding affinity to FcγRIIIa and FcyRIIa compared to conventional variants (FIGS. 18 and 19).

## Claims

1. A human antibody Fc domain variant in which one or more amino acids selected from the group consisting of amino acids at positions 249, 252, 268, 276, 326, 384, 392, 422, 435 and 436 numbered according to a Kabat numbering system in a wild-type human antibody Fc domain are substituted with sequences different from wild-type amino acids.

2. The human antibody Fc domain variant of claim 1, wherein the human antibody Fc domain variant comprises any one or more amino acid substitutions selected from the group consisting of 249N, 252I, 276N, 268Y, 276K, 326N, 384N, 384S, 392K, 392N, 422V, 422I, 435H, 435R, 436Y and 436F.

3. The human antibody Fc domain variant of claim 1, wherein the antibody is immunoglobulin (IgG).

4. The human antibody Fc domain variant of claim 1, wherein the antibody is IgG1 or IgG3 isotypes.

5. The human antibody Fc domain variant of claim 1, wherein the human antibody Fc domain variant comprises any one or more amino acid substitutions selected from the group consisting of N276K, N384S, K392N, and V422I in a wild-type human IgG1 Fc domain.

6. The human antibody Fc domain variant of claim 1, wherein the human antibody Fc domain variant comprises any one or more amino acid substitutions selected from the group consisting of D249N, M252I, H268Y, K276N, K326N, S384N, N392K, I422V, R435H, and F436Y in a wild-type human IgG3 Fc domain.

7. The human antibody Fc domain variant of claim 1, wherein the binding affinity to C1q is increased compared to a wild-type human antibody Fc domain.

8. The human antibody Fc domain variant of claim 1, wherein an effector function is increased compared to the wild-type human antibody Fc domain.

9. The human antibody Fc domain variant of claim 8, wherein the effector function is an Fc-mediated effector function selected from C1q-binding, complement activation, complement dependent cytotoxicity (CDC), antibody-dependent cellular cytotoxicity (ADCC), Fc-receptor binding including Fc-gamma receptor binding, protein A-binding, protein G-binding, antibody-dependent cell-mediated phagocytosis (ADCP), complement dependent cell-mediated cytotoxicity (CDCC), complement-enhanced cytotoxicity, opsonization, Fc-containing polypeptide internalization, target downmodulation, ADC uptake, induction of apoptosis, cell death, cell cycle arrest, and any combination thereof.

10. The human antibody Fc domain variant of claim 1, wherein the complement-dependent cytotoxicity (CDC) is increased compared to the wild-type human antibody Fc domain.

11. The human antibody Fc domain variant of claim 1, wherein the human antibody Fc domain variant comprises a heavy chain constant region domain 2 (C_{H}2) comprising any one selected from the group consisting of amino acid sequences represented by SEQ ID NOS: 1 to 8 and a heavy chain constant region domain 3 (C_{H}3) comprising any one selected from the group consisting of amino acid sequences represented by SEQ ID NOS: 9 to 16.

12. An antibody or immunologically active fragment thereof, comprising the human antibody Fc domain variant of claim 1.

13. The antibody or immunologically active fragment thereof of claim 12, wherein the binding affinity to C1q is increased compared to a wild-type human antibody.

14. The antibody or immunologically active fragment thereof of claim 12, wherein the antibody is a polyclonal antibody, a monoclonal antibody, a minibody, a domain antibody, a bispecific antibody, an antibody mimetic, a chimeric antibody, an antibody conjugation, a human antibody or a humanized antibody.

15. The antibody or immunologically active fragment thereof of claim 12, wherein the antibody is an anti-human epidermal growth factor receptor 2 (HER2) antibody, an anti-epidermal growth factor receptor (EGFR) antibody, or an anti-CD20 antibody.

16. The antibody or immunologically active fragment thereof of claim 12, wherein the antibody is trastuzumab, cetuximab or rituximab.

17. A nucleic acid molecule encoding the human antibody Fc domain variant of claim 1, or the antibody or immunologically active fragment thereof of claim 12.

18. A vector comprising the nucleic acid molecule of claim 17.

19. A host cell comprising the vector of claim 18.

20. A pharmaceutical composition for preventing or treating cancer comprising the human antibody Fc domain variant of claim 1, or the antibody or immunologically active fragment thereof of claim 12 as an active ingredient.

21. The pharmaceutical composition for preventing or treating cancer of claim 20, wherein the cancer is any one selected from the group consisting of brain tumor, melanoma, myeloma, non-small cell lung cancer, oral cancer, liver cancer, gastric cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas, and pituitary adenomas.

22. A method for preparing a human antibody Fc domain variant comprising:
a) incubating a host cell comprising a vector including a nucleic acid molecule encoding the human antibody Fc domain variant of claim 1; and
b) recovering a polypeptide expressed by the host cell.

23. A use of an antibody or immunologically active fragment thereof comprising the human antibody Fc domain variant of claim 1 to be used in the preparation of an antibody therapeutic agent.

24. A use of an antibody or immunologically active fragment thereof comprising the human antibody Fc domain variant of claim 1 for the prevention or treatment of cancer.

25. A method for treating cancer comprising administering an antibody or immunologically active fragment thereof comprising the human antibody Fc domain variant of claim 1 in a pharmaceutically effective amount to a subject suffering from cancer.
